(19)

Europäisches
Patentamt
European
Patent Office
Office européen
des brevets

(11) **EP 2 151 494 A2**

(12) **EUROPEAN PATENT APPLICATION**

(43) Date of publication:
**10.02.2010 Bulletin 2010/06**

(51) Int Cl.:
*C12N 9/28* (2006.01)      *C12N 15/56* (2006.01)
*C12P 7/06* (2006.01)      *C12P 19/12* (2006.01)
*A21D 8/04* (2006.01)

(21) Application number: **09173277.6**

(22) Date of filing: **22.07.2005**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **02.08.2004   DK 200401173**

(62) Document number(s) of the earlier application(s) in
accordance with Art. 76 EPC:
**05762147.6 / 1 797 179**

(71) Applicant: **Novozymes A/S
2880 Bagsvaerd (DK)**

(72) Inventors:
• **Beier, Lars
2800, Lyngby (DK)**
• **Svendsen, Allan
2970, Hørsholm (DK)**
• **Borchert, Torben, Vedel
3460, Birkerød (DK)**

Remarks:
This application was filed on 16-10-2009 as a
divisional application to the application mentioned
under INID code 62.

(54)   **Maltogenic alpha-amylase variants**

(57)   The inventors realized that in some applications the control of the maltose-to-glucose ratio is of great importance. Particularly for ethanol production from granular starch by fermentation, it may be an advantage to form a larger amount of glucose which is more readily fermentable than maltose. Particularly for production of maltose syrups glucose is an undesired product, and hence it of interest to increase the maltose-to-glucose ratio. They then developed a method of constructing such variants of based on the three-dimensional structure of a parent maltogenic alpha-amylase.

EP 2 151 494 A2

SEQUENCE LISTING

<110> Novozymes A/S

<120> Maltogenic alpha-amylase variants

<130> 10573-204

<160> 1

<170> PatentIn version 3.2

<210> 1
<211> 686
<212> PRT
<213> Bacillus stearothermophilus

<400> 1

```
Ser Ser Ser Ala Ser Val Lys Gly Asp Val Ile Tyr Gln Ile Ile Ile
1               5                   10                  15

Asp Arg Phe Tyr Asp Gly Asp Thr Thr Asn Asn Asn Pro Ala Lys Ser
            20                  25                  30

Tyr Gly Leu Tyr Asp Pro Thr Lys Ser Lys Trp Lys Met Tyr Trp Gly
            35                  40                  45

Gly Asp Leu Glu Gly Val Arg Gln Lys Leu Pro Tyr Leu Lys Gln Leu
        50                  55                  60

Gly Val Thr Thr Ile Trp Leu Ser Pro Val Leu Asp Asn Leu Asp Thr
65                  70                  75                  80

Leu Ala Gly Thr Asp Asn Thr Gly Tyr His Gly Tyr Trp Thr Arg Asp
                85                  90                  95

Phe Lys Gln Ile Glu Glu His Phe Gly Asn Trp Thr Thr Phe Asp Thr
                100                 105                 110

Leu Val Asn Asp Ala His Gln Asn Gly Ile Lys Val Ile Val Asp Phe
            115                 120                 125

Val Pro Asn His Ser Thr Pro Phe Lys Ala Asn Asp Ser Thr Phe Ala
        130                 135                 140

Glu Gly Gly Ala Leu Tyr Asn Asn Gly Thr Tyr Met Gly Asn Tyr Phe
145                 150                 155                 160
```

```
Asp Asp Ala Thr Lys Gly Tyr Phe His His Asn Gly Asp Ile Ser Asn
             165                 170                 175

Trp Asp Asp Arg Tyr Glu Ala Gln Trp Lys Asn Phe Thr Asp Pro Ala
             180                 185                 190

Gly Phe Ser Leu Ala Asp Leu Ser Gln Glu Asn Gly Thr Ile Ala Gln
             195                 200                 205

Tyr Leu Thr Asp Ala Ala Val Gln Leu Val Ala His Gly Ala Asp Gly
    210                 215                 220

Leu Arg Ile Asp Ala Val Lys His Phe Asn Ser Gly Phe Ser Lys Ser
225                 230                 235                 240

Leu Ala Asp Lys Leu Tyr Gln Lys Lys Asp Ile Phe Leu Val Gly Glu
             245                 250                 255

Trp Tyr Gly Asp Asp Pro Gly Thr Ala Asn His Leu Glu Lys Val Arg
             260                 265                 270

Tyr Ala Asn Asn Ser Gly Val Asn Val Leu Asp Phe Asp Leu Asn Thr
             275                 280                 285

Val Ile Arg Asn Val Phe Gly Thr Phe Thr Gln Thr Met Tyr Asp Leu
    290                 295                 300

Asn Asn Met Val Asn Gln Thr Gly Asn Glu Tyr Lys Tyr Lys Glu Asn
305                 310                 315                 320

Leu Ile Thr Phe Ile Asp Asn His Asp Met Ser Arg Phe Leu Ser Val
             325                 330                 335

Asn Ser Asn Lys Ala Asn Leu His Gln Ala Leu Ala Phe Ile Leu Thr
             340                 345                 350

Ser Arg Gly Thr Pro Ser Ile Tyr Tyr Gly Thr Glu Gln Tyr Met Ala
    355                 360                 365

Gly Gly Asn Asp Pro Tyr Asn Arg Gly Met Met Pro Ala Phe Asp Thr
    370                 375                 380

Thr Thr Thr Ala Phe Lys Glu Val Ser Thr Leu Ala Gly Leu Arg Arg
```

385              390              395              400

Asn Asn Ala Ala Ile Gln Tyr Gly Thr Thr Thr Gln Arg Trp Ile Asn
            405              410                415

Asn Asp Val Tyr Ile Tyr Glu Arg Lys Phe Phe Asn Asp Val Val Leu
            420              425              430

Val Ala Ile Asn Arg Asn Thr Gln Ser Ser Tyr Ser Ile Ser Gly Leu
            435              440              445

Gln Thr Ala Leu Pro Asn Gly Ser Tyr Ala Asp Tyr Leu Ser Gly Leu
            450              455              460

Leu Gly Gly Asn Gly Ile Ser Val Ser Asn Gly Ser Val Ala Ser Phe
465              470              475              480

Thr Leu Ala Pro Gly Ala Val Ser Val Trp Gln Tyr Ser Thr Ser Ala
            485              490              495

Ser Ala Pro Gln Ile Gly Ser Val Ala Pro Asn Met Gly Ile Pro Gly
            500              505              510

Asn Val Val Thr Ile Asp Gly Lys Gly Phe Gly Thr Thr Gln Gly Thr
            515              520              525

Val Thr Phe Gly Gly Val Thr Ala Thr Val Lys Ser Trp Thr Ser Asn
            530              535              540

Arg Ile Glu Val Tyr Val Pro Asn Met Ala Ala Gly Leu Thr Asp Val
545              550              555              560

Lys Val Thr Ala Gly Gly Val Ser Ser Asn Leu Tyr Ser Tyr Asn Ile
            565              570              575

Leu Ser Gly Thr Gln Thr Ser Val Val Phe Thr Val Lys Ser Ala Pro
            580              585              590

Pro Thr Asn Leu Gly Asp Lys Ile Tyr Leu Thr Gly Asn Ile Pro Glu
            595              600              605

Leu Gly Asn Trp Ser Thr Asp Thr Ser Gly Ala Val Asn Asn Ala Gln
            610              615              620

```
Gly Pro Leu Leu Ala Pro Asn Tyr Pro Asp Trp Phe Tyr Val Phe Ser
625             630             635                 640


Val Pro Ala Gly Lys Thr Ile Gln Phe Lys Phe Phe Ile Lys Arg Ala
                645             650                 655


Asp Gly Thr Ile Gln Trp Glu Asn Gly Ser Asn His Val Ala Thr Thr
            660             665                 670


Pro Thr Gly Ala Thr Gly Asn Ile Thr Val Thr Trp Gln Asn
        675             680                 685
```

**Description**

**FIELD OF THE INVENTION**

The invention relates to the preparation of variants of a parent maltogenic alpha-amylase, where hydrolysis products of said variants having a modified of maltose-to-glucose ratio as compared to hydrolysis products of the parent maltogenic alpha-amylase. It also relates to a polynucleotide encoding such variants and to the use of the variants in the production of ethanol, beer, dough, maltose syrup and baked products.

**BACKGROUND OF THE INVENTION**

Maltogenic alpha-amylase (EC 3.2.1.1) is known to be useful, e.g., for production of ethanol from granular starch by fermentation (WO 2003068976) and for retarding the staling of bread (WO 9104669). One maltogenic alpha-amylase is the commercial product Novamyl® described in EP 120693 B1. Variants of Novamyl are known from WO 9943794. Maltogenic alpha-amylases are known to hydrolyze starch with formation of maltose as the main product together with a minor amount glucose.

**SUMMARY OF THE INVENTION**

The inventors realized that in some applications the control of the maltose-to-glucose ratio is of great importance. Particularly for ethanol production from granular starch by fermentation, it may be an advantage to form a larger amount of glucose which is more readily fermentable than maltose. Particularly for production of maltose syrups glucose is an undesired product, and hence it of interest to increase the maltose-to-glucose ratio. They then developed a method of constructing such variants of based on the three-dimensional structure of a parent maltogenic alpha-amylase.

Accordingly, the invention provides a method of constructing a variant polypeptide, comprising:

a) providing a parent maltogenic alpha-amylase having an amino acid sequence and a three-dimensional structure which includes a cleavage point and a substrate with at least three monosaccharide moieties at the reducing side of the cleavage point,
b) selecting an amino acid residue having a C-alpha atom located <10 Å from an atom in the substrate,
c) substituting or deleting the selected residue to obtain a modified amino acid sequence,
d) preparing a polypeptide having the modified sequence,
e) testing the modified polypeptide by incubating it with starch and analyzing the reaction product, and
f) selecting a modified polypeptide which has the ability to hydrolyze starch and wherein the hydrolysis product has a modified maltose-to-glucose ratio compared to an hydrolysis product made with the parent maltogenic alpha-amylase.

The parent maltogenic alpha-amylase and the substrate may for the purpose of steps a), b), and c) be provided in the form of a computer model.
The invention also provides a variant polypeptide which

a) has an amino acid sequence having more than 80 % identity to SEQ ID NO: 1,
b) compared to SEQ ID NO: 1 has a different amino acid residue at a position corresponding to W93, T134, G172, N176, D178, F188, D190, D198, I227 V230, K231, H232, F233, Y258, G259, D260, D261, P262, T264, N266, F284, T288 or M330 or a deletion corresponding to 191-195, and
c) has the ability to hydrolyze starch to form an product having a modified maltose-to-glucose ratio than a product made with the polypeptide of SEQ ID NO: 1.

Finally, the invention provides a polynucleotide encoding the polypeptide and uses of the polypeptide in production of ethanol from granular starch by fermentation, in production of maltose syrup, and in the production of dough and baked products.

**DETAILED DESCRIPTION OF THE INVENTION**

**Maltogenic alpha-amylase**

The maltogenic alpha-amylase (EC 3.2.1.133) may have the amino acid sequence shown in SEQ ID NO: 1 (in the following referred to as Novamyl) with a 3D structure including a substrate as described in US 6162628 and found in the Protein Data Bank with the identifier 1QHO. Alternatively, the maltogenic alpha-amylase may be a Novamyl variant described in US 6162628. A 3D structure of such a variant may be developed from the Novamyl structure by known methods, e.g. as described in T.L. Blundell et al., Nature, vol. 326, p. 347 ff (26 March 1987); J. Greer, Proteins: Structure, Function and Genetics, 7:317-334 (1990); or Example 1 of WO 9623874.

**Selection of residues**

An amino acid residue is selected which has a C-alpha atom located <10 Å from an atom of the substrate. In 1qho, the following residues are selected by this criterion: 13, 15, 18, 43-44, 70, 72-73, 77-78, 82, 86-94, 97, 127-136, 143, 174-180, 183-184, 187-198, 226-233, 255-267, 270, 282-289, 291-292, 299, 307, 324, 327-331, 360, 370-376.

The selection may in particular be for residues <10 Å from an atom in monosaccharide (glucose) moieties +1, +2 and +3 at the reducing side of the cleavage point. In 1qho, the moieties are denoted j, k and l, and this lead to selection of the following residues: 13, 70, 73, 90, 92-93, 127-132, 174-180, 183-184, 187-191, 196, 226-233, 255-267, 270, 282-289, 291-292, 299, 307, 324, 327-331, 371-372, 375-376.

**Amino acid substitutions**

The selected residue may be substituted so as to push the substrate away or block for it presents in position +1, +2 and +3 etc by making the residues larger at a position corresponding to G172, D178, T189, K231, H232, Y258, G259, D260, T264, N266 or T288 in Novamyl (SEQ ID NO: 1), e.g. a substitution corresponding to G172V, T189M, K231R, H232Y, Y258W, G259A/H/Y, T264Y/Q/F, N266Y or T288Y/Q/F/P.

The substitution may serve to remove hydrogen bonding or van der Waals contact to the substrate at position +1, +2 and +3. This may be done by substituting with a smaller residue at a position corresponding to W93, T134, D178, D190, D198, I227, K231, H232, F233, Y258, D260, D261, T264 or T288 of SEQ ID NO: 1, particularly a substitution corresponding to W93S/G/V/T/M/E, T134A, D178L/M/T/V, D190G, D198G, I227V, K231L/M, H232L/M, F233S, Y258L/M/T/V, D260L/M/T/V, D261G, T264A/V or T288A/V.

Alternatively, a hydrophilic or electrically charged (positive or negative) residue may be substituted with a hydrophobic residue, particularly at a position corresponding to T134, D178, D190, D198, K231, H232 or D261, more particularly a substitution corresponding to T134A, D178V, D190G, D198G, K231L/M, H232L/M or D261G.

Finally, the substitution or deletion may serve to change indirectly the contact by changing the residues next to the substrate contact residues, particularly a residue corresponding to W93, N176, 191, 192, 193, 194,195, V230, P262, F284 or M330 in Novamyl, e.g. a substitution corresponding to W93E/G/M/V/T/S, N176L, V230G, F284Y or M330I or a deletion of residues corresponding to 191, 192, 193, 194, and 195.

Amino acid residues are ranked as follows from smallest to largest: (an equal sign indicates residues with sizes that are practically indistinguishable):

$$G < A=S=C < V=T < P < L=I=N=D=M < E=Q < K < H < R < F < Y < W$$

The following amino acid residues are considered to be hydrophobic: G, A, V, L, I, P, F, W and C as part of a disulfide bridge.

**Variants**

Some particular variants according to the invention have the sequence of SEQ ID NO: 1 with the following substitutions:

| |
|---|
| W93M |
| W93E |
| W93M, V230G |
| Y258W |
| Y258W, F284Y |
| H232M |
| F188T |

(continued)

| |
|---|
| F188G |
| F188V |
| W93G |
| W93V |
| W93T |
| W93S |
| N176L |
| D178V |
| F188G, W93M |
| F188G, W93E |
| F188G, W93S |
| F188G, W93T |
| F188V, W93M |
| F188V, W93E |
| F188V, W93S |
| F188V, W93T |

**Ability to hydrolyze starch**

The variant of the invention is able to hydrolyze starch to form a product having a modified maltose-to-glucose ratio as compared to a product made with the polypeptide of SEQ ID NO: 1. The starch hydrolysis may be carried out by the following procedures described in the examples. The variants of the invention may show an increased ratio of glucose to maltose (DP1/DP2) or an increased ratio of DP1/(DP1-4) or an increased ratio of maltose to glucose (DP2/DP1) or an increased ratio of (DP1-4)/DP1.

Starch is in the context of the present invention intended to include starch as well as breakdown products of starch, such as amylopectin, or amylose, or maltooligosaccharides.

**Amino acid identity**

The polypeptide of the invention may have identities to the disclosed sequences of at least 80 %, particularly at least 85 % or at least 90 %, e.g. at least 91%, or 92%, or 93%, or 94%, or at least 95 %, such as 96%, or 97%, or 98%, or 99%.

For purposes of the present invention, alignments of sequences and calculation of identity scores may be done using a Needleman-Wunsch alignment (i.e. global alignment), useful for both protein and DNA alignments. The default scoring matrices BLOSUM50 and the identity matrix are used for protein and DNA alignments respectively. The penalty for the first residue in a gap is -12 for proteins and -16 for DNA, while the penalty for additional residues in a gap is -2 for proteins and -4 for DNA. Alignment is from the FASTA package version v20u6 (W. R. Pearson and D. J. Lipman (1988), "Improved Tools for Biological Sequence Analysis", PNAS 85:2444-2448, and W. R. Pearson (1990) "Rapid and Sensitive Sequence Comparison with FASTP and FASTA", Methods in Enzymology, 183:63-98).

**Industrial uses**

The variant of the invention may be used in various known applications for amylases, e.g. production of ethanol, beer, dough, maltose syrup and baked products.

Ethanol production

The variant may be used in a process comprising treating granular starch with the variant and fermentation into ethanol. The treatment of the granular starch serves to produce a hydrolysis product which includes a significant

4

amount of glucose. The fermentation to produce ethanol may be simultaneous with the granular starch treatment, or the starch may first be hydrolyzed followed by fermentation of the hydrolysate. The process may be performed as described in WO 2003068976.

Beer production

The variant may be used in mashing, i.e. in the process of converting starch from milled malt and solid adjuncts into fermentable and unfermentable sugars to produce wort. The mashing involves incubating the variant with milled malt and solid adjuncts in water to hydrolyze the starch.

Dough and baked products

The variant may be added to dough for making baked products such as bread. Addition of the variant may serve to retard staling of the baked product. The addition to dough may be done as described in WO 9104669.

Maltose syrup

The variant may be used for commercial production of maltose, which today starts from liquefied starch (DE<10), which is subsequently treated simultaneously with debranching enzymes (pullulanase or isoamylase) and maltose-forming enzymes (maltogenic α-amylase or β-amylase) at a temperature around 60°C. Glucose is an undesired side product in maltose syrups because it impacts the crystallization of maltose. Maltose is used in large quantities as syrups in e.g. the confectionary industry and as a sweetening agent in the food industry. Maltose syrups have among other capacities reduced browning capacity, a resistance to moisture absorption and to crystallization making maltose syrups suited for e.g. frozen dessert formulations, hard candy, jams, and jellies. Thus, a maltogenic alpha-amylase with an increased maltose-to-glucose ratio would be an advantage in the production of maltose syrups.

**EXAMPLES**

**Example 1: Starch hydrolysis with variants**

A number of variants were prepared, each having the sequence of SEQ ID NO: 1 with the indicated substitutions. Each variant was tested by incubating it with maltodextrin (DE 11) by application of the following procedure:

- Prepare a 30% (w/w) maltodextrin solution (DE 11) in 50mM Na-acetate, 1mM CaCl2 pH 5.5. Is heated to 60°C for dissolving the maltodextrins.
- 1ml substrate is added to 1.5 ml tubes with lid and membrane, and samples are preheated to 60°C on a thermomixer.
- 1-100 microliter fermentation broth was added to 1ml preheated substrate. The fermentation broth volumes were adjusted thus, that they all contained the same amount of amylase activity measured by the Phadebas amylase assay.
- Samples are incubated for 42h at 60°C.
- Make a small hole in the lid with e.g. a needle.
- Samples are boiled for 15 minutes (or 99°C at the thermomixer).
- Add 1ml Milli-Q.
- After cooling the samples are filtered through a 0.2 micro-m filter.

The carbohydrate profile was determined by chromatography by applying standard procedures, e.g. as described in Norman,B.E. in James N.Bemiller, David J.Manners, and Robert J.Sturgeon (eds), Methods in Carbohydrate Chemistry, Volume X. John Wiley & Sons, Inc., New York, pp. 231-239, 1994.
Novamyl without substitutions was included as reference. The results were as follows:

| Substitutions | % glucose | % maltose | Maltose/glucose ratio |
|---|---|---|---|
| Novamyl parent | 4-5 | 50-55 | 11 |
| W93M | 10 | 42 | 4.2 |
| W93E | 10 | 33 | 3.3 |
| W93M, V230G | 12 | 42 | 3.5 |
| Y258W | 8 | 45 | 5.6 |

(continued)

| Substitutions | % glucose | % maltose | Maltose/glucose ratio |
|---|---|---|---|
| Y258W, F284Y | 8 | 22 | 2.8 |
| H232M | 6 | 22 | 3.7 |
| F188T | 20 | 51 | 2.6 |
| F188G | 20 | 44 | 2.2 |
| F188V | 15 | 55 | 3.7 |
| W93G | 13 | 52 | 4.0 |
| W93V | 13 | 36 | 2.8 |
| W93T | 12 | 37 | 3.1 |
| W93S | 8 | 36 | 4.5 |
| W93T,F188V | 14 | 27 | 1.9 |
| N176L | 11 | 50 | 4.5 |
| D178V | 12 | 51 | 4.3 |
| N26S, L51M, T80A, F237L, N266Y, M330I | 10 | 42 | 4.2 |
| d(191-195)[1], D261 G, T288P | 8 | 21 | 2.6 |
| W185R, D198G, E202V | 16 | 52 | 3.3 |
| T134A, H170R, D190G, V215A, F233S, I251T | 4 | 14 | 3.5 |
| G172V, D178V, G204D | 11 | 45 | 4.1 |
| R55C, K137M, 288S, S331 P, 396V | 4.5 | 52.5 | 11.7 |
| N176Y, E202D | 3.5 | 43.1 | 12.3 |
| T189M, A219V | 4.1 | 49.8 | 12.1 |
| T189M,A214T,F237L,T288S | 3.2 | 41.6 | 13.1 |
| D161G,N176Y,T189M,N203D,A214T | 3.4 | 45.4 | 13.3 |
| A148D,T189M,A219V | 3.6 | 47.2 | 13.1 |
| T189M,Q208R,A219V,D657G | 4.0 | 50.2 | 12.5 |
| F104L,N106D,K137M,D173N,N176Y,T189M, E202D,V254A, L334, P380L,G512D,Y632C | 3.3 | 54.7 | 16.7 |
| K137M,T189M,S195T,E202D,G263R,S331P, A388V,N631 S | 3.7 | 57.8 | 15.5 |
| H103R,T189M,I227V,K239R,V254A,T288S, S441P,Y460H, F649L | 2.4 | 38.8 | 15.9 |
| 1) d (191-195) indicates a deletion of the amino acids corresponding to position 191, 192, 193, 194, and 195. | | | |

**Example 2: Starch hydrolysis with variants and pullulanase**

Further a number of variants were tested applying the same procedure as described in Example 1, except that 1.2 mg/g DS of the commercially available pullulanase Promozyme® (EP 63909) was added.
The following results were obtained:

| Substitutions | % glucose | % maltose | Maltose/glucose ratio |
|---|---|---|---|
| Parent Novamyl | 7.2 | 71.6 | 9.9 |
| A148D,T189M,G263R,N337D,Y572C, F636L | 5.9 | 69.8 | 11.9 |

(continued)

| Substitutions | % glucose | % maltose | Maltose/glucose ratio |
|---|---|---|---|
| D173N,N176Y,T189M,A219V,Y246H,T288S,L33 4P,N631S, K650R | 5.5 | 70.6 | 12.8 |
| N27S,T80I,T189M,S195T,E202D,I290V,T386A,L 596P | 4.9 | 67.6 | 13.7 |
| A148D,T189M,D212G,A219V,T288S | 5.9 | 69.9 | 11.9 |
| K137M,N158Y,N176H,T189M,E202D,V254A, S331P,A388V | 5.1 | 63.8 | 12.4 |

**Example 3: Starch hydrolysis with purified variants and pullulanase**

A number of purified variants (each having the sequence of SEQ ID NO: 1 with the indicated substitutions) were prepared by standard purification techniques, see e.g. Beier et al.: "Conversion of the maltogenic alpha-amylase Novamyl into a CGTase" in Protein Engineering, vol. 13 no. 7 pp. 509-513, 2000.

Each variant was tested by incubating it with maltodextrin (DE 11) at 60°C and pH 5.5 for 42 hours as described in Example 1. Either an amount of 0.81 micro g (variants marked with [1]) or 1.62 micro g (variants marked with [2]) of the variant was added, and further 1.2 mg/g DS of the commercially available pullulanase Promozyme® (EP 63909) was added.

Novamyl without substitutions was included as reference. The results were as follows:

| Substitutions | % maltose | % glucose | Maltose/glucose ratio |
|---|---|---|---|
| Novamyl parent [1] | 56.7 | 5.5 | 10.3 |
| Novamyl parent [2] | 66.1 | 6.6 | 10.1 |
| Y258W [1] | 32.0 | 5.5 | 5.8 |
| Y258W [2] | 37.3 | 6.8 | 5.5 |
| W93S [1] | 40.4 | 11.2 | 3.6 |
| W93S [2] | 46.0 | 14.2 | 3.2 |
| T189M,A214T,F237L,T288S [1] | 56.6 | 5.0 | 11.3 |
| T189M,A214T,F237L,T288S [2] | 63.0 | 5.8 | 10.9 |
| D161G,N176Y,T189M,N203D,A214T [1] | 48.1 | 3.8 | 12.7 |
| D161G,N176Y,T189M,N203D,A214T [2] | 61.3 | 5.2 | 11.8 |
| A148D,T189M,A219V [1] | 54.6 | 4.4 | 12.4 |
| A148D,T189M,A219V [2] | 63.4 | 5.4 | 11.7 |
| T189M,Q208R,A219V,D657G [1] | 52.8 | 4.4 | 12.0 |
| T189M,Q208R,A219V,D657G [2] | 65.7 | 5.9 | 11.1 |
| F104L,N106D,K137M, D173N,N176Y,T189M, E202D,V254A,L334P, P380L,G512D,Y632C [1] | 35.0 | 2.0 | 17.5 |
| F104L,N106D,K137M,D173N,N176Y,T189M, E202D,V254A,L334P,P380L,G512D, Y632C [2] | 40.6 | 2.3 | 17.7 |
| K137M,T189M,S195T,E202D,G263R,S331 P, A388V,N631S [1] | 59.9 | 3.8 | 15.8 |
| K137M,T189M,S195T,E202D,G263R,S331P, A388V,N631S [2] | 67.9 | 4.6 | 14.8 |

(continued)

| Substitutions | % maltose | % glucose | Maltose/glucose ratio |
|---|---|---|---|
| H103R,T189M,1227V,K239R,V254A,T288S, S441P,Y460H,F649L [1] | 36.7 | 2.5 | 15.0 |
| H103R,T189M,1227V,K239R,V254A,T288S, S441 P,Y460H,F649L [2] | 68.2 | 4.8 | 14.2 |

**Example 4: Starch hydrolysis with purified variants without addition of pullulanase**

A number of purified variants (each having the sequence of SEQ ID NO: 1 with the indicated substitutions) were prepared by standard purification techniques, see e.g. Beier et al.: "Conversion of the maltogenic alpha-amylase Novamyl into a CGTase" in Protein Engineering, vol. 13 no. 7 pp. 509-513, 2000.

Each variant was tested by incubating it with maltodextrin (DE 11) at 60°C and pH 5.5 for 42 hours as described in Example 1. An amount 1.62 micro g (variants marked with [2]) of the variant was added.

A single variant was dosed at a higher amount, namely 38.2 micro g (variant marked with [3]).

| Substitutions | % maltose | % glucose | Maltose/glucose ratio |
|---|---|---|---|
| Parent Novamyl [2] | 57.8 | 5.2 | 11.0 |
| F188G [3] | 46.2 | 26.3 | 1.8 |
| T189M,A214T,F237L,T288S [2] | 53.4 | 4.4 | 12.1 |
| D161G,N176Y,T189M, N203D, A214T [2] | 57.7 | 4.7 | 12.3 |
| A148D,T189M,A219V [2] | 56.4 | 4.4 | 12.7 |
| T189M,Q208R,A219V, D657G [2] | 55.5 | 4.5 | 12.3 |

**Example 5: Amylopectin hydrolysis with variants**

A number of purified variants (each having the sequence of SEQ ID NO: 1 with the indicated substitutions) were prepared by standard purification techniques, see e.g. Beier et al.: "Conversion of the maltogenic alpha-amylase Novamyl into a CGTase" in Protein Engineering, vol. 13 no. 7 pp. 509-513, 2000.

Each variant was tested by incubating it with amylopectin (waxy maize starch) by application of the following procedure:

- Prepare a 5% (w/w) amylopectin solution in 50mM Na-acetate, 1mM CaCl2, pH 5.5. The solution is boiled for 2 minutes or until it is dissolved.
- 1mL substrate is added to 1.5 mL tubes with lid and membrane, and samples are preheated to 60°C on a thermomixer.
- Enzyme is dosed at a dose corresponding to the amylase activity of 0.81 micro g Novamyl measured by the Phadesbas amylase assay (which is 0.045 PSU/mL substrate, when Novamyl activity is 56.8PSU/mg)
- Samples are incubated for1 hour or 24 hours at 60°C.
- 1ml Milli-Q is added with 1-2 drops of 1M HCl (pH must be less than 3 to inactive the amylase).
- Make a small hole in the lid with e.g. a needle.
- Samples are boiled for 15 minutes.
- After cooling the samples are filtered through a 0.2 micro m filter.

Samples incubated for 1 hour are marked [1], samples incubated for 24 hours are marked [24].

The carbohydrate profile was determined by chromatography by applying standard procedures, e.g. as described in Norman,B.E. in James N.Bemiller, David J.Manners, and Robert J.Sturgeon (eds), Methods in Carbohydrate Chemistry, Volume X. John Wiley & Sons, Inc., New York, pp. 231-239, 1994.

Novamyl without substitutions was included as reference. The results were as follows:

| Substitutions | % maltose | % glucose | Maltose/glucose ratio |
|---|---|---|---|
| Novamyl parent [1] | 53.2 | 1.5 | 35.1 |
| Novamyl parent [24] | 64.3 | 2.6 | 24.7 |
| Y258W [1] | 39.1 | 2.7 | 14.4 |
| Y258W [24] | 57.6 | 4.1 | 13.9 |
| F188G [1] | 12.3 | 0.0 | - |
| F188G [24] | 17.4 | 2.4 | 7.2 |
| W93S [1] | 13.3 | 1.8 | 7.5 |
| W93S [24] | 52.7 | 15.3 | 3.4 |
| F104L,N106D,K137M,D173N, N176Y,T189M,E202D,V254A, L334P,P380L,G512D,Y632C [1] | 49.9 | 1.2 | 41.6 |
| F104L,N106D,K137M,D173N, N 176Y,T189M,E202D,V254A, L334P,P380L,G512D,Y632C [24] | 66.9 | 1.5 | 44.6 |
| K137M,T189M,S195T,E202D, G263R,S331P,A388V,N631S [1] | 66.2 | 0.6 | 110 |
| K137M,T189M,S195T,E202D, G263R,S331 P,A388V,N631 S [24] | 64.4 | 0.8 | 80.5 |
| H103R,T189M,1227V,K239R, V254A,T288S,S441P,Y460H, F649L [1] | 54.0 | 0.9 | 60.0 |
| H103R,T189M,1227V,K239R, V254A,T288S,S441 P,Y460H, F649L [24] | 63.0 | 1.3 | 48.5 |

## Example 6: Baking with variants

Two variants were tested for baking, namely Y258W and W93S. Bread was made by the European Straight Dough method with and without addition of enzymes. The texture was evaluated using standard AACC procedures, and the following results were obtained after 7 days storage:

| Firmness (g) | | |
|---|---|---|
| Reference: no enzyme | Y258W | W93S |
| 1600 | 1250 | 1250 |
| Elasticity % (g/g) | | |
| Reference: no enzyme | Y258W | W93S |
| 50 | 53 | 54 |
| Free water mobility (micro S) | | |
| Reference: no enzyme | Y258W | W93S |

(continued)

| Free water mobility (micro S) | | |
|---|---|---|
| 10600 | 10850 | 11100 |
| Y258W was dosed 5 mg enzyme protein/kg flour for all three tests. W93S was dosed 3 mg enzyme protein/kg flour for all three tests. | | |

**Claims**

1. A polypeptide which

   a) has an amino acid sequence having more than 80% identity to SEQ ID NO: 1,
   b) compared to SEQ ID NO: 1 has a different amino acid residue at a position corresponding to W93, T134, G172, N176, D178, F188, D190, D198, I227, V230, K231, H232, F233, Y258, G259, D260, D261, P262, T264, N266, F284, T288 or M330 or a deletion corresponding to 191-195, and
   c) has the ability to hydrolyze starch to form a product having a modified maltose-to-glucose ratio than a product made with the polypeptide of SEQ ID NO: 1.

2. The polypeptide of the preceding claim, which compared to SEQ ID NO: 1 comprises a substitution corresponding to W93E/G/M/V/T/S, T134A, G172V, D178L/M/T/V, F188G/T/V, D190G, D198G, I227V, V230G, K231 R/L/M, 232Y/L/M, F233S, Y258W/L/M/T/V, G259A/H/Y, D260L/M/T/V, D261 G, T264Y/Q/F/A/V, N266Y, F284Y, T288Y/Q/F/A/V/P or M330I.

3. The polypeptide of claim 2 or 3, which has the amino acid sequence of SEQ ID NO: 1 with the following alterations:

| |
|---|
| W93M |
| W93E |
| W93M, V230G |
| Y258W |
| Y258W, F284Y |
| H232M |
| F188T |
| F188G |
| F188V |
| W93G |
| W93V |
| W93T |

(continued)

| |
|---|
| W93S |
| N176L |
| D178V |
| F188G, W93M |
| F188G, W93E |
| F188G, W93S |
| F188G, W93T |
| F188V, W93M |
| F188V, W93E |
| F188V, W93S |
| F188V, W93T |

4. A polynucleotide encoding the polypeptide of any preceding claim.

5. A process for the production of ethanol, comprising treating granular starch with the polypeptide of any preceding claim and fermentation into ethanol.

6. A dough comprising the polypeptide of any preceding claim.

7. A method of producing dough or a baked product from dough, comprising adding the polypeptide of any preceding claim.

8. A process for the production of maltose syrup comprising treating liquefied starch with the polypeptide of any preceding claim.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2003068976 A
- WO 9104669 A
- EP 120693 B1
- WO 9943794 A
- US 6162628 A
- WO 9623874 A
- EP 63909 A

### Non-patent literature cited in the description

- **T.L. Blundell et al.** *Nature,* 26 March 1987, vol. 326, 347 ff
- **J. Greer.** *Proteins: Structure, Function and Genetics,* 1990, vol. 7, 317-334
- **W. R. Pearson ; D. J. Lipman.** Improved Tools for Biological Sequence Analysis. *PNAS,* 1988, vol. 85, 2444-2448
- **W. R. Pearson.** Rapid and Sensitive Sequence Comparison with FASTP and FASTA. *Methods in Enzymology,* 1990, vol. 183, 63-98
- **Norman,B.E.** Methods in Carbohydrate Chemistry. John Wiley & Sons, Inc, 1994, vol. X., 231-239
- **Beier et al.** Conversion of the maltogenic alpha-amylase Novamyl into a CGTase. *Protein Engineering,* 2000, vol. 13 (7), 509-513